# EUROPEAN PATENT APPLICATION

(11) **EP 4 840 425 A1**
(43) Date of publication of application: **18.11.2026**
(21) Application number: 26175219.0
(22) Date of filing: 28.04.2026
(51) Int. Cl.: A61F 5/01

(54) **TOGGLE-LINK KNEE JOINT SUPPORT AND ASSIST BRACE**

(30) Priority: 13.05.2025 TW 114117875
(71) Applicant: Liao, Yen-Ting, Taichung City 407040 (TW)
(72) Inventor: LIAO, SU-CHANG, 407 Taichung City (TW)
(74) Representative: Cabinet Chaillot

(57) **Abstract**

A toggle-link knee joint support and assist brace includes a thigh bracket having a main arm body and a first fixing seat, a lower leg bracket having a restraining arm and a second fixing seat, a pivot shaft interconnecting the two fixing seats, and a power-assist actuator therebetween. The power-assist actuator includes a flexible, fluid-tight bladder body defining at least one fluid chamber, a toggle-link skeleton on the exterior of the bladder body, an actuating link coupled to the first fixing seat, a restraining link coupled to the second fixing seat, and a fluid connector communicating with the fluid chamber. The bladder body forms deformable sections each corresponding to a toggle link including a first toggle bar and a second toggle bar. Fluid pressure causing expansion or contraction of the bladder body is converted, via the toggle-link skeleton, into a supporting or assist-pushing force between the two fixing seats.

## Description

### 1. Fields of the invention

The present invention relates to a toggle-link knee joint support and assist brace, and more particularly to a technology applied in the medical field that provides patients with knee joint injuries a device for rehabilitation or therapeutic training. During rehabilitation and treatment, the brace maintains flexible walking motion, cushioned support during squatting, and an assist-pushing function when rising from a squatting position.

### 2. Descriptions of Related Art

The knee joint is one of the most important joints in the human body, bearing substantial load and participating in activities such as walking, standing, and squatting. Its structure includes bones such as the femur, tibia, and patella, as well as cartilage, menisci, ligaments, and tendons. Because the knee joint bears a significant load and moves frequently during daily activities, it is also one of the joints most susceptible to injury. Common knee joint injuries include anterior cruciate ligament tears, meniscal injuries, and degenerative osteoarthritis. During the rehabilitation period following treatment or surgery for such injuries, external support devices are typically required to help stabilize the knee joint, avoid excessive loading, and facilitate the rehabilitation process.

Accordingly, various knee joint braces are currently available on the market. Such braces typically include an upper bracket and a lower bracket respectively secured to the thigh and lower leg of the user, with a pivoting structure positioned at the knee joint to allow relative rotation between the upper and lower brackets. In addition, an angle-limiting mechanism is commonly provided at the pivot location to restrict the range of motion of the knee joint, thereby preventing excessive bending or improper movement during rehabilitation. Straps or other fastening members are used to secure the upper and lower brackets to the user's leg to provide a degree of mechanical support during activity.

However, the aforementioned conventional knee joint braces primarily provide protection by limiting the range of joint motion, and their functions are largely confined to providing support and restricting movement. When the user performs movements such as squatting or standing up, the knee joint still bears a significant load, and the brace itself is generally incapable of providing additional assist force. Consequently, in certain rehabilitation scenarios, the user may still need to rely on a handrail or other external support when rising from a squatting or seated position. Furthermore, long-term reliance on braces that merely provide fixation and restriction may diminish the user's motivation to actively engage in muscle-strengthening exercises, thereby adversely affecting rehabilitation efficiency.

Therefore, in the field of knee joint rehabilitation and assistive support, there remains a need for further improvement of the knee joint brace structure in order to enhance the supporting effect and convenience of use during daily activities and the rehabilitation process.

The present invention intends to provide a toggle-link knee joint support and assist brace to eliminate shortcomings mentioned above.

### SUMMARY OF THE INVENTION

The primary object of the present invention is to provide a knee joint brace that, while maintaining basic support and range-of-motion restriction, is capable of delivering adjustable assist force or assistive support to the user when needed, thereby improving convenience in daily activities and rehabilitation efficiency, and remedying the deficiencies cited in the prior art.

To achieve the aforementioned effects and remedy the deficiencies of the prior art, the present invention provides a toggle-link knee joint support and assist brace, and comprises a thigh bracket having a main arm body and a first fixing seat formed at a bottom end thereof, the thigh bracket being securable to a human thigh. A lower leg bracket has a restraining arm and a second fixing seat formed at an upper end thereof. The lower leg bracket is supportable against a human lower leg. A pivot shaft interconnects the first fixing seat and the second fixing seat. A power-assist actuator is disposed between the first fixing seat and the second fixing seat.

The power-assist actuator comprises a flexible and fluid-tight bladder body. The interior of the bladder body defines at least one fluid chamber. The bladder body has an actuation end and a restraint end located respectively at opposite ends thereof. The bladder body forms a plurality of deformable sections between the actuation end and the restraint end. The deformable sections are interconnected and allow the at least one fluid chamber to expand or contract as fluid flows in or out.

A toggle-link skeleton is disposed on the exterior of the bladder body, and the toggle-link skeleton comprises a plurality of mutually pivoted toggle links. Each toggle link is formed by pivotally connecting a first toggle bar and a second toggle bar. Each first toggle bar is pivotally connected to an adjacent second toggle bar. Each first toggle bar and each second toggle bar correspond to a respective deformable section so as to support and guide the deformation of the bladder body.

An actuating link connects one end of the toggle-link skeleton and is coupled to the first fixing seat. A restraining link connects the other end of the toggle-link skeleton and is coupled to the second fixing seat. At least one fluid connector is disposed on the actuating link or the restraining link and in fluid communication with the at least one fluid chamber of the bladder body. When the bladder body expands or contracts under fluid pressure, the deformation is converted, through the arrangement of the toggle-link skeleton, into a supporting or assist-pushing force between the first fixing seat and the second fixing seat.

The present invention will become more obvious from the following description when taken in connection with the accompanying drawings which show, for purposes of illustration only, a preferred embodiment in accordance with the present invention.

### BRIEF DESCRIPTION OF THE DRAWINGS

FIG. 1 is a perspective view of the present invention;
FIG. 2 is an exploded perspective view showing the thigh bracket, the lower leg bracket, the pivot shaft, and the power-assist actuator of the present invention;
FIG. 3 is a perspective view of the power-assist actuator of the present invention;
FIG. 4 is an exploded perspective view of FIG. 3;
FIG. 5 is an exploded perspective view of the toggle-link skeleton of FIG. 4;
FIG. 6 is an exploded perspective view of the bladder body of FIG. 4;
FIG. 7 is a cross-sectional view taken along line VII-VII of FIG. 1;
FIG. 8 is a partially enlarged view of FIG. 7;
FIG. 9 is a schematic view showing the brace worn on a human body in an upright position;
FIG. 10 is a partially enlarged schematic view of FIG. 9, showing fluid freely flowing in and out of the fluid chamber;
FIG. 11 is a circuit diagram showing fluid freely flowing in and out of the fluid chamber in a hydraulic fluid supply system embodiment of the present invention;
FIG. 12 is a circuit diagram showing fluid being discharged from the fluid chamber in the hydraulic fluid supply system embodiment of the present invention;
FIG. 13 is a circuit diagram showing fluid freely flowing in and out of the fluid chamber in a pneumatic fluid supply system embodiment of the present invention;
FIG. 14 is a circuit diagram showing fluid being discharged from the fluid chamber in the pneumatic fluid supply system embodiment of the present invention;
FIG. 15 is a schematic view showing the state during fluid discharge from the fluid chamber of the power-assist actuator of FIG. 10;
FIG. 16 is a schematic view showing the state after the user wearing the brace has sat down on a chair;
FIG. 17 is a schematic view showing the fluid discharge state in the fluid chamber corresponding to the seated position of FIG. 16;

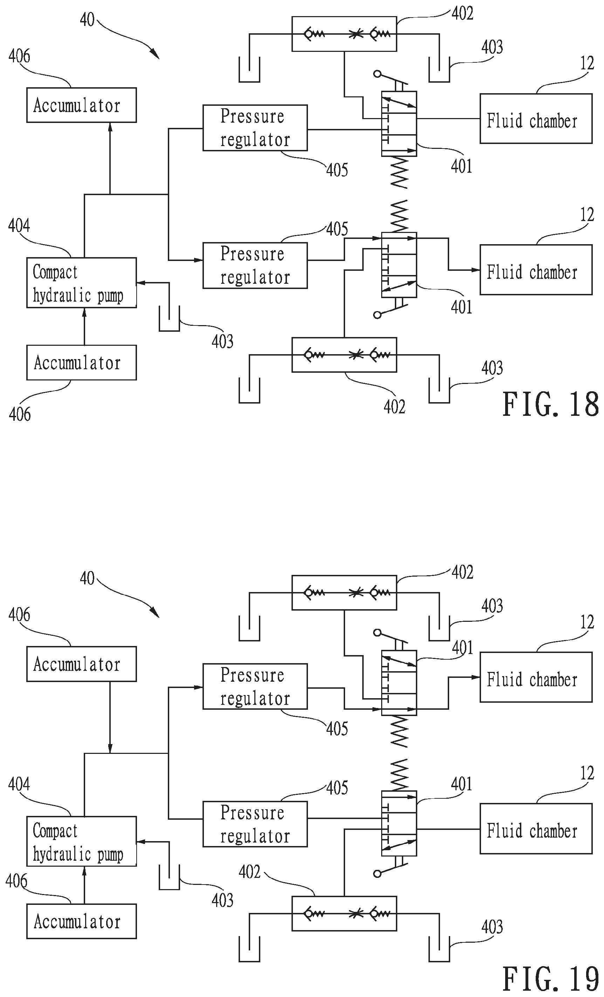

FIG. 18 is a circuit diagram showing pressurization of one of two fluid chambers in the hydraulic fluid supply system embodiment of the present invention;
FIG. 19 is a circuit diagram showing pressurization of the other one of the two fluid chambers in the hydraulic fluid supply system embodiment of the present invention;

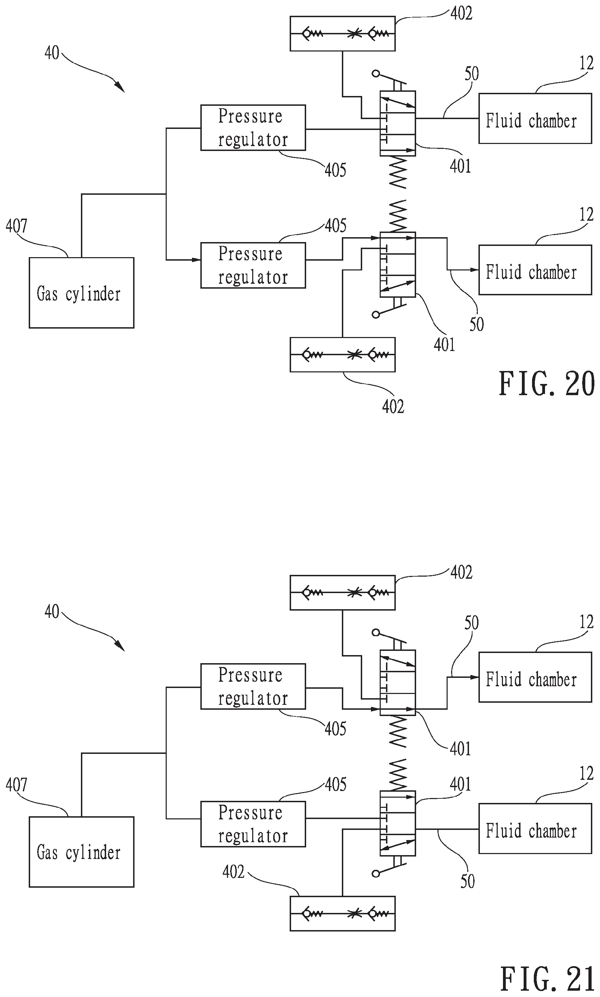

FIG. 20 is a circuit diagram showing pressurization of one of two fluid chambers in the pneumatic fluid supply system embodiment of the present invention;
FIG. 21 is a circuit diagram showing pressurization of the other one of the two fluid chambers in the pneumatic fluid supply system embodiment of the present invention;
FIG. 22 is a schematic view showing pressurization of one of two fluid chambers within the power-assist actuator of the present invention;
FIG. 23 is a schematic view of FIG. 22 showing pressurization of the other fluid chamber within the power-assist actuator;

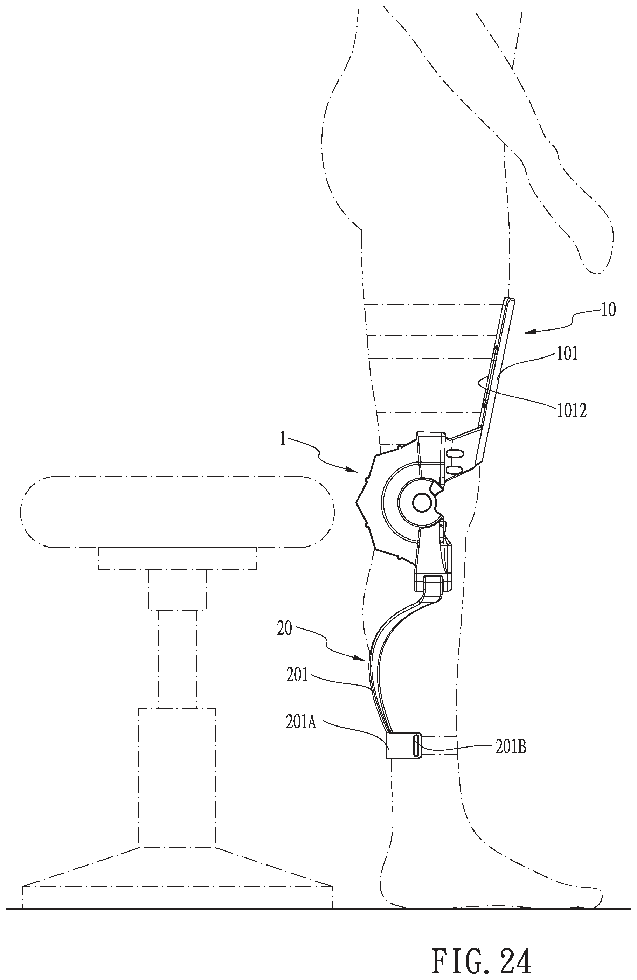

FIG. 24 is a schematic view showing the power-assist actuator of FIG. 16 assisting the user in rising to a standing position;
FIG. 25 is a perspective view of the bladder body with a U-shaped retainer secured thereto;
FIG. 26 is an exploded perspective view of the bladder body and the U-shaped retainer of FIG. 25;
FIG. 27 is a cross-sectional view taken along line XXVII-XXVII of FIG. 25;
FIG. 28 is a schematic view of FIG. 27 as applied in the power-assist actuator, with the U-shaped retainer secured to the actuator and restraining the toggle pivot points;
FIG. 29 is a perspective view of the bladder body covered by a fabric sleeve;
FIG. 30 is a cross-sectional view taken along line XXX-XXX of FIG. 29;
FIG. 31 is a cross-sectional view of the assembled power-assist actuator structure when applied as in FIG. 29;
FIG. 32 is a schematic view of FIG. 31 showing the fabric sleeve on the power-assist actuator limiting the expansion angle of the first and second toggle bars;
FIG. 33 is an external perspective view of FIG. 29 with U-shaped retainers mounted thereon;
FIG. 34 is a cross-sectional view taken along line XXXIV-XXXIV of FIG. 33;
FIG. 35 is a cross-sectional view of the assembled power-assist actuator structure when applied as in FIG. 34, and
FIG. 36 is a schematic view of FIG. 35, showing the power-assist actuator with a fabric sleeve and U-shaped retainers limiting the expansion angle of the first and second toggle bars and restraining the toggle pivot points.

### DETAILED DESCRIPTION OF THE PREFERRED EMBODIMENT

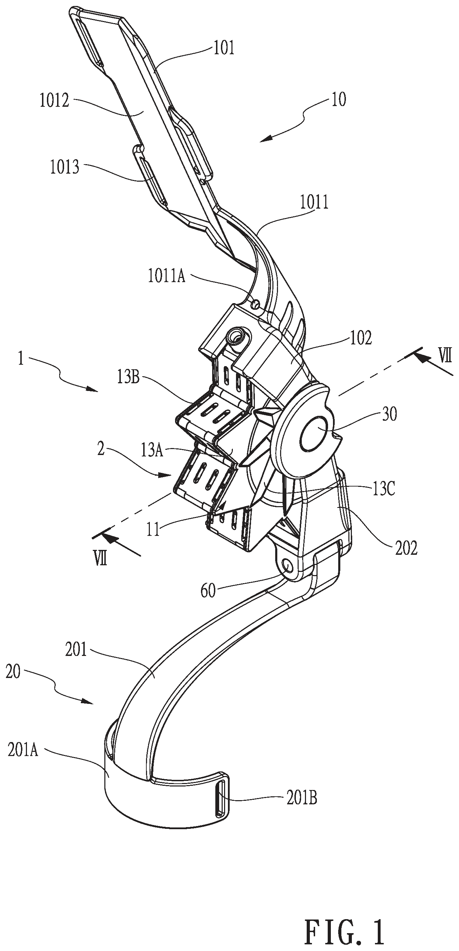

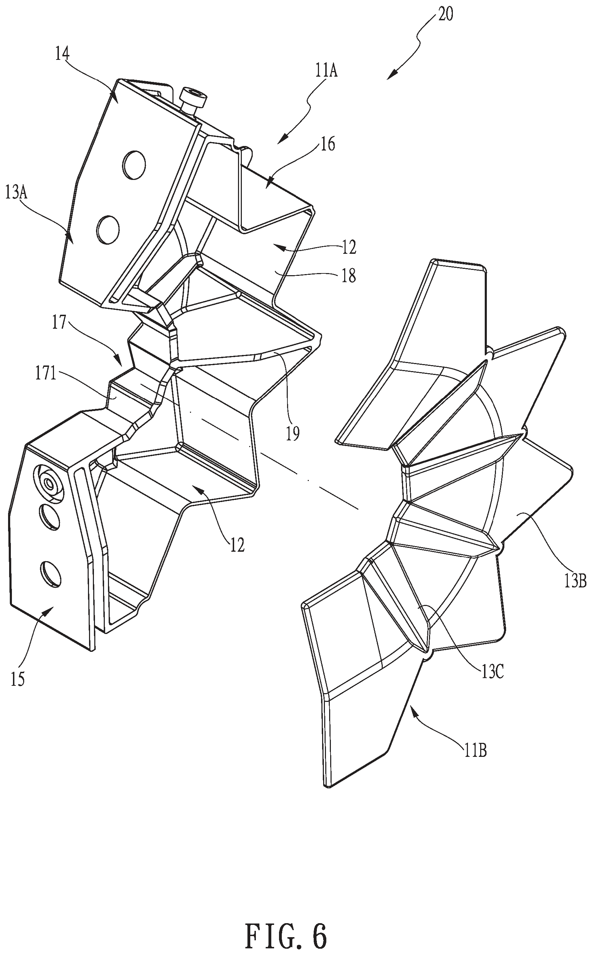

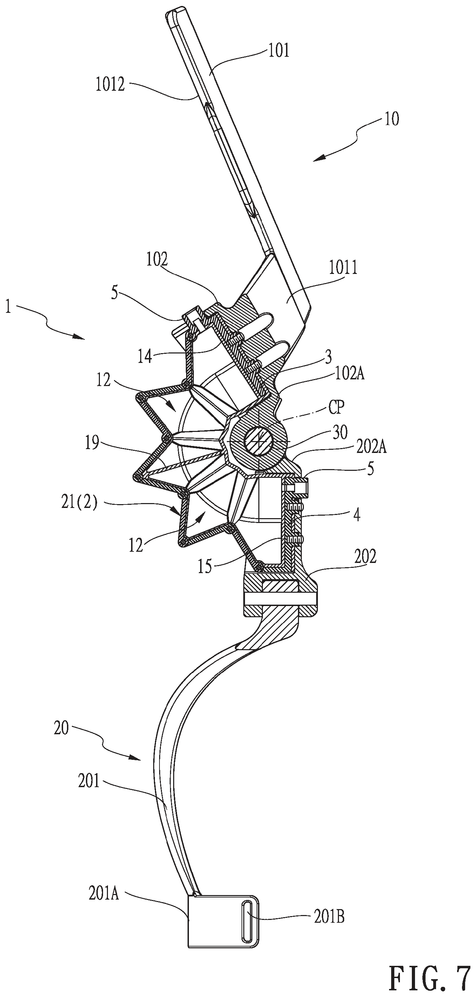

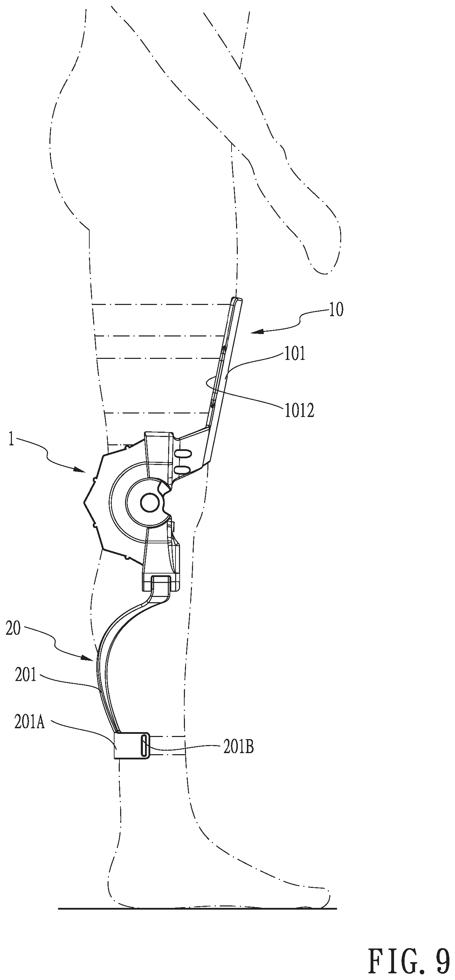

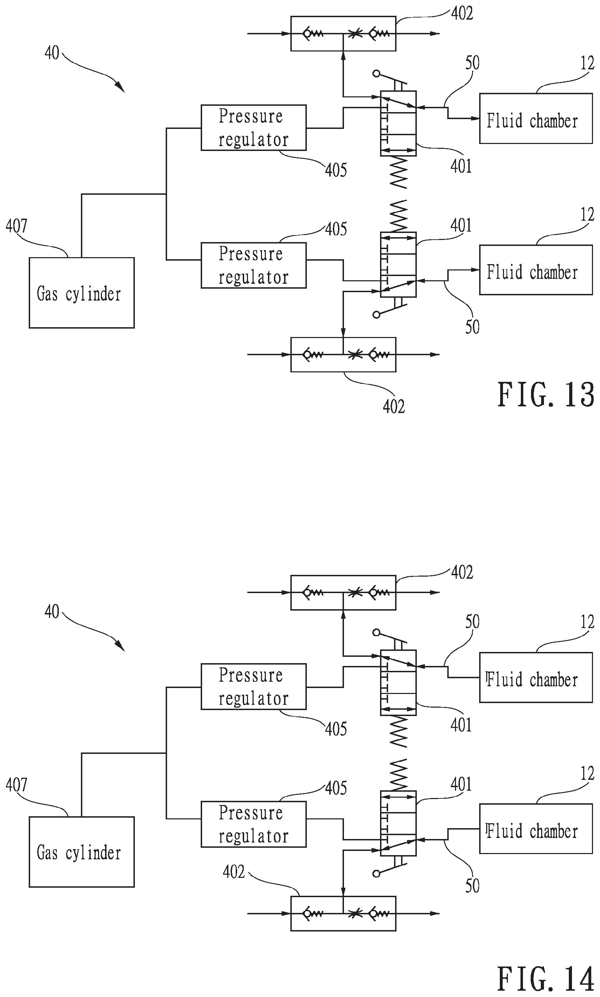

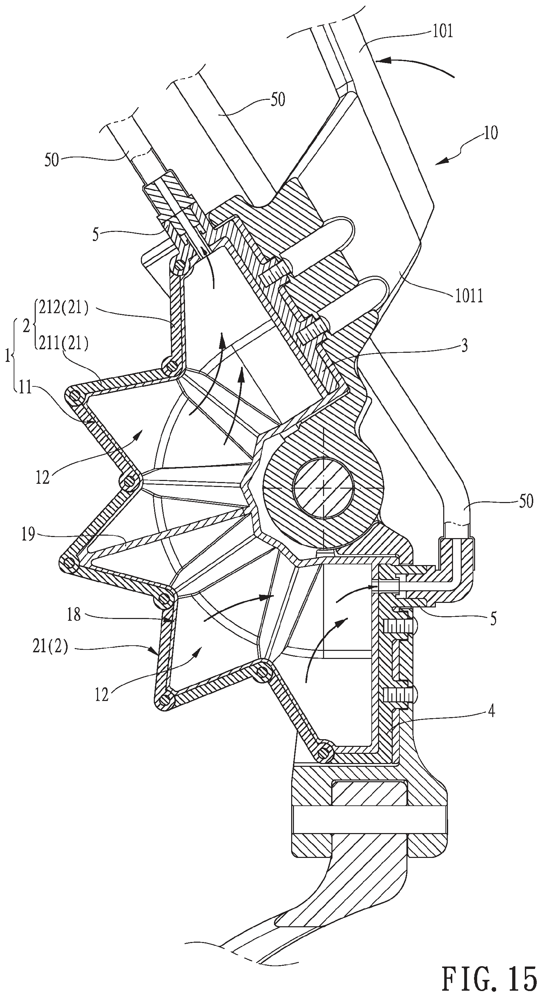

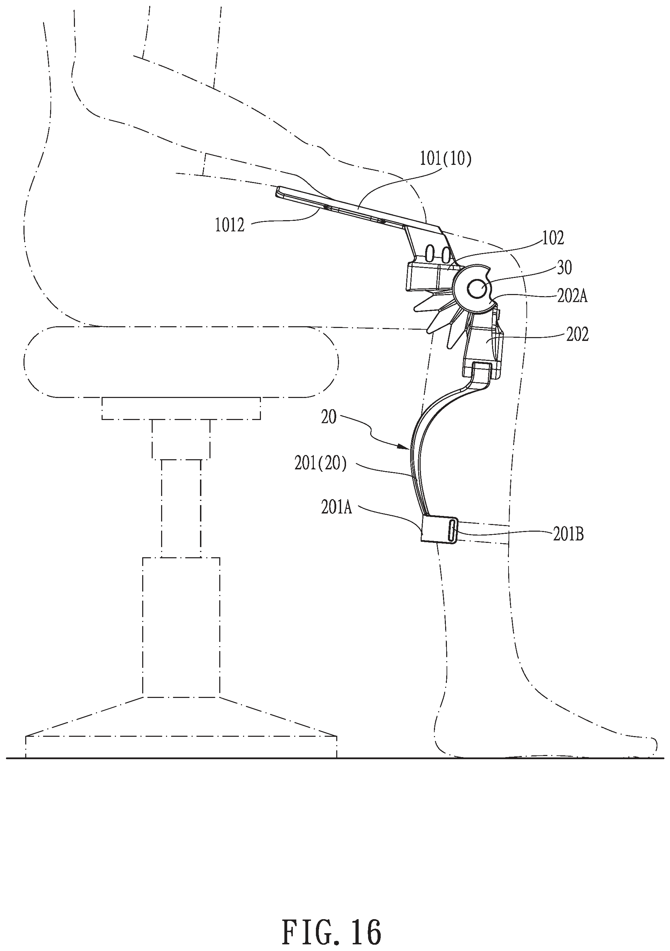

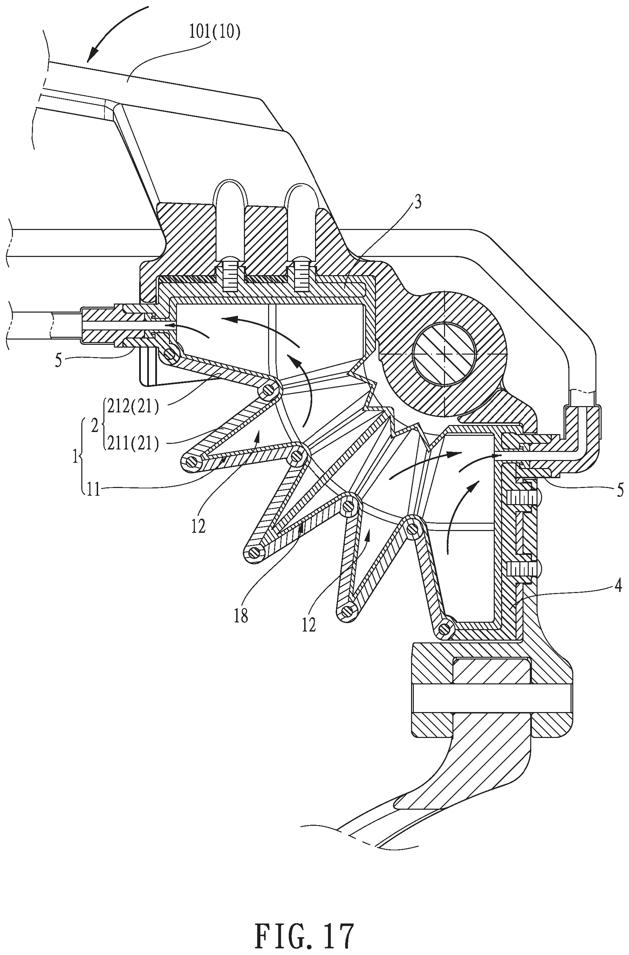

Referring to FIGS. 1 through 17, the toggle-link knee joint support and assist brace of the present invention comprises a thigh bracket (10), which is an arm-shaped frame having structural rigidity (formed from metal or non-metallic rigid plastic material). The thigh bracket (10) has a main arm body (101) and a first fixing seat (102) formed at the bottom end of the main arm body (101). The main arm body (101) is adapted to be secured to a user's thigh by straps, and the first fixing seat (102) is formed at the front end of a connecting portion (1011) at the bottom end of the main arm body (101), presenting a curved configuration (adapted to the curved profile of the human thigh, but ideally configured as a frame-shaped seat).

A lower leg bracket (20), which is an arm member having tensile strength (formed from metal or non-metallic rigid plastic material). The lower leg bracket (20) has a restraining arm (201) and a second fixing seat (202) formed at the upper end of the restraining arm (201) (ideally configured as a frame-shaped seat), and is adapted to be supported against a user's lower leg. The bottom end of the restraining arm (201) is formed with a supporting rest portion adapted to rest non-fixedly against the user's lower leg using straps.

A pivot shaft (30) interconnects the first fixing seat (102) and the second fixing seat (202).

A power-assist actuator (1) is disposed between the first fixing seat (102) and the second fixing seat (202).

The power-assist actuator (1) comprises a flexible and fluid-tight bladder body (11). The bladder body (11) has a fan-shaped curved configuration and is formed from a fluid-tight material (which may be rubber or silicone, or a material composed of resilient natural or synthetic fibers laminated with a soft adhesive or film), constituting a flexible, compliant bladder member that permits expansion and contraction. The interior of the bladder body (11) defines at least one fluid chamber (12). The bladder body (11) has an actuation end (14) and a restraint end (15) located respectively at the two lateral faces (13A, 13B) of the bladder body (11). The peripheries of the two lateral faces (13A, 13B) share a common center point (CP) between the actuation end (14) and the restraint end (15), defining an outer peripheral surface (16) and an inner peripheral surface (17). The bladder body (11) forms a plurality of deformable sections (18) between the actuation end (14) and the restraint end (15). The deformable sections (18) are interconnected, allowing the at least one fluid chamber (12) to expand or contract as fluid flows in or out. The junctions between adjacent deformable sections (18) present a continuous V-shaped corrugated profile on the outer peripheral surface (16), and on the inner peripheral surface (17) present a pleated surface (171) formed by V-shaped folds that permit opening and folding.

A toggle-link skeleton (2) is disposed on the outer peripheral surface (16) of the bladder body (11). The toggle-link skeleton (2) comprises a plurality of mutually pivoted toggle links (21). Each toggle link (21) is formed by pivotally connecting a first toggle bar (211) and a second toggle bar (212). Each first toggle bar (211) is pivotally connected to an adjacent second toggle bar (212). Each first toggle bar (211) and each second toggle bar (212) correspond to a respective deformable section (18) so as to support and guide the deformation of the bladder body (11). Each toggle link (21) is composed of a first toggle bar (211) and a second toggle bar (212) that have structural rigidity (made of metal or rigid plastic), pivotally connected to each other in a V-shaped configuration and stacked together on a respective one of the deformable sections (18).

An actuating link (3) and a restraining link (4) are both rigid link members having structural strength (made of metal or rigid plastic). The actuating link (3) connects one end of the toggle-link skeleton (2) and is coupled to the first fixing seat (102), with the actuating link (3) connected to the actuation end (14). The restraining link (4) connects the other end of the toggle-link skeleton (2) and is coupled to the second fixing seat (202), with the restraining link (4) stacked at the restraint end (15).

At least one fluid connector (5) is disposed on either the actuating link (3) or the restraining link (4) and is in fluid communication with the at least one fluid chamber (12) of the bladder body (11). When the bladder body (11) expands or contracts under fluid pressure, the deformation is converted, through the arrangement of the toggle-link skeleton (2), into a supporting or assist-pushing force between the first fixing seat (102) and the second fixing seat (202). The at least one fluid connector (5) is provided with a channel (51) communicating with the at least one fluid chamber (12), allowing fluid within the at least one fluid chamber (12) to flow in and out through the at least one fluid connector (5).

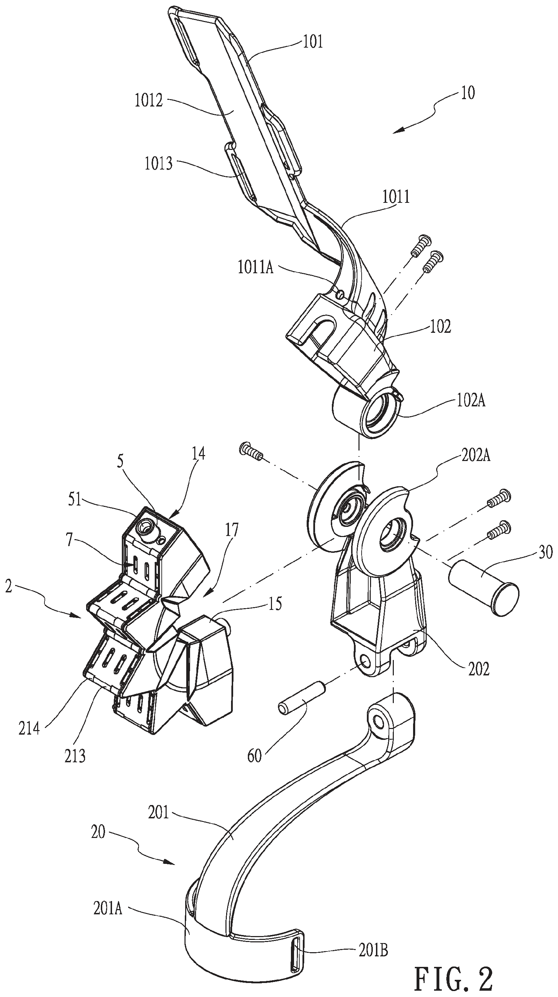

Based on the foregoing description and referring to FIGS. 2, 4, and 11 through 17, at one end of the bottom side of the first fixing seat (102), a first pivot hole seat (102A) is formed about the center point (CP). Correspondingly, on one side of the second fixing seat (202), a second pivot hole seat (202A) is formed in alignment with the first pivot hole seat (102A). The pivot shaft (30) passes through the first pivot hole seat (102A) and the second pivot hole seat (202A) so that the first pivot hole seat (102A) and the second pivot hole seat (202A) are pivotally connected to each other.

When the at least one fluid connector (5) receives fluid (either liquid or gaseous) from a fluid supply system (40) to pressurize the fluid within the fluid chamber (12) of the power-assist actuator (1), the expanded fluid chamber (12) causes each deformable section (18) to expand and deform, which in turn causes the V-shaped included angle between the first toggle bar (211) and the second toggle bar (212) of each toggle link (21) to open. This generates a thrust force with accumulated energy that is concentrated, using the restraining link (4) as a stationary support, onto the actuating link (3), causing the main arm body (101) of the thigh bracket (10) to pivot outwardly about the pivot shaft (30) in response to the thrust from the actuating link (3), thereby transmitting the thrust force generated by the power-assist actuator (1) to the main arm body (101). This thrust action is defined as the assist-pushing action of the power-assist actuator (1).

Conversely, when an external force (such as the force of the thigh during squatting) acts on the main arm body (101), causing the main arm body (101) to pivot inwardly (counter clockwise in the drawings), the actuating link (3) causes the V-shaped included angle between the first toggle bar (211) and the second toggle bar (212) of each toggle link (21) to decrease, which in turn compresses each deformable section (18) into a folded state, compressing and reducing the volume of the fluid chamber (12). As the fluid chamber (12) volume decreases, the fluid therein is expelled through the at least one fluid connector (5) toward the fluid supply system (40). The fluid discharge action from the fluid chamber (12) as the main arm body (101) pivots inwardly is defined as the cushioning action of the power-assist actuator (1).

Accordingly, by setting (switching) the fluid supply system (40) so that fluid in the fluid chamber (12) can flow freely in and out, the user may install the knee joint brace on the thigh and lower leg while in either a standing or seated position. Under this condition, during normal walking, the flow of fluid in and out of the fluid chamber (12) within the bladder body (11) is regulated, providing flexible protection and limitation to the movement of the knee joint. Similarly, during the squatting or sitting-down process and upon reaching the seated position, the regulated fluid discharge from the fluid chamber (12) enables the cushioning action generated by the power-assist actuator (1) to flexibly attenuate excess stress on the myofascial tissues of the knee joint; the magnitude of the cushioning support can be adjusted by controlling the fluid discharge flow rate to correspond to the requirements of the knee joint rehabilitation or training regimen.

Furthermore, when the user rises from a squatting or seated position, the fluid supply system (40) pressurizes the fluid in the fluid chamber (12) of the bladder body (11), causing the assist-pushing action generated by the power-assist actuator (1) to lift (drive) the thigh via the main arm body (101) of the thigh bracket (10), assisting the knee joint in exerting force so that the user can perform or complete the act of standing up without the aid of a handrail or external support device. The magnitude of the assist-pushing force can be adjusted by controlling the pressurization level of the fluid to correspond to the requirements of the knee joint rehabilitation or training regimen. The overall knee joint brace structure uses the toggle-link skeleton (2) on the bladder body (11) as the energy-generating and energy-transmitting element for the power-assist actuator (1) during both the cushioning action and the assist-pushing action, enabling the knee joint brace to provide solid cushioning and assist-pushing energy for the rehabilitation training of patients with knee joint injuries-effectively achieving flexible protection of the knee joint during walking, flexible attenuation of knee joint loading during squatting, and assist-pushing of the knee joint during rising from a seated or squatting position.

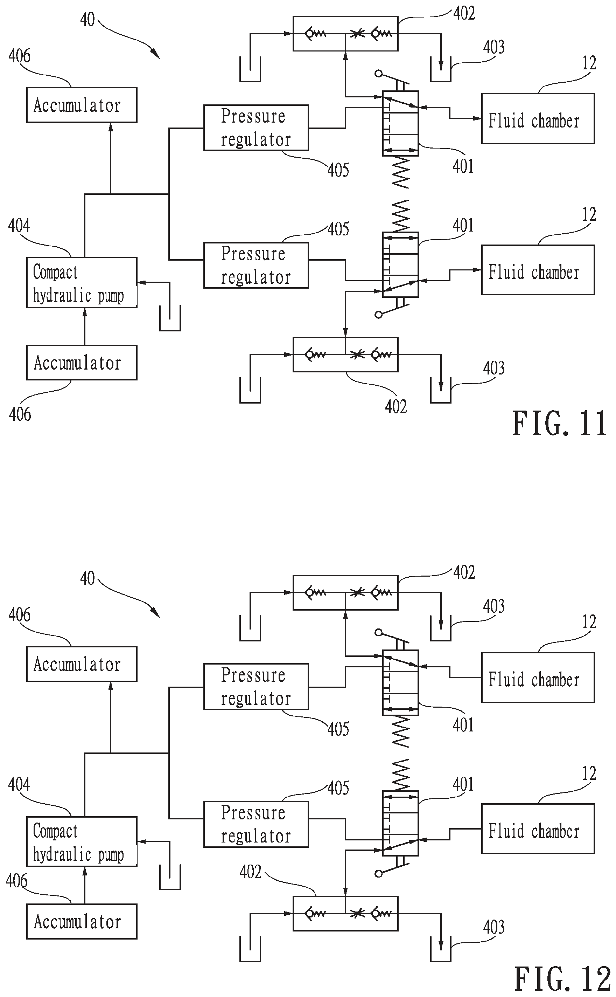

Referring to FIGS. 11 through 14, the fluid supply system (40) may be integrated and worn by the user on a backpack or belt (not shown). The fluid supply system (40) may be a hydraulic fluid supply system, in which a manually or electrically controlled switching valve (401) switches (or sets) the liquid fluid in the fluid chamber (12) of the power-assist actuator (1) to communicate (allowing free fluid ingress/egress) with a reservoir or oil tank (403) via a flow controller (402). Through flow regulation (or restriction) by the flow controller (402), the power-assist actuator (1) generates a supporting or restrictive cushioning action on the movement of the knee joint. When an assist-pushing action is required, the switching valve (401) is actuated to route hydraulic fluid from a compact hydraulic pump (404) through a pressure regulator (405) to pressurize the fluid chamber (12). The compact hydraulic pump (404) may, during periods when no assist-pushing action is needed (idle periods), direct fluid into an accumulator (406) for storage. When needed, the hydraulic fluid is released from the accumulator (406) to meet the demand for the assist-pushing action. Alternatively, a manually controlled switching valve (401) may switch the gaseous fluid in the fluid chamber (12) of the power-assist actuator (1) to communicate with the atmosphere (allowing free gas ingress/egress) via the flow controller (402). Through flow regulation (or restriction) by the flow controller (402), the power-assist actuator (1) generates a supporting or restrictive cushioning action on the movement of the knee joint. When an assist-pushing action is required, the switching valve (401) is actuated to route pneumatic fluid released from a gas cylinder (407) (such as a CO₂ cylinder used in paintball guns) through the pressure regulator (405) to pressurize the fluid chamber (12) with compressed gas, causing the power-assist actuator (1) to generate an assist-pushing action.

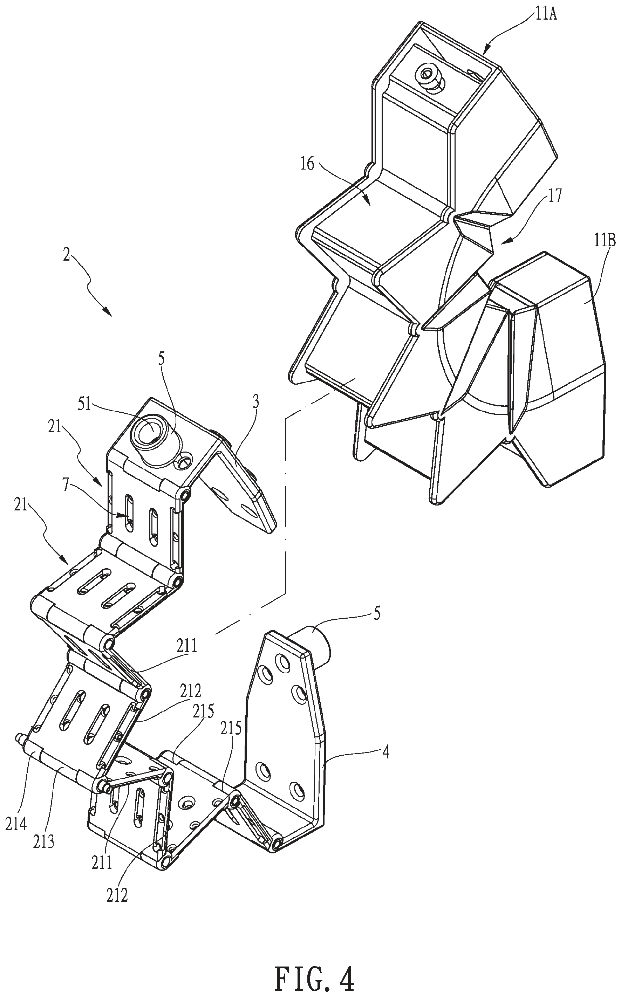

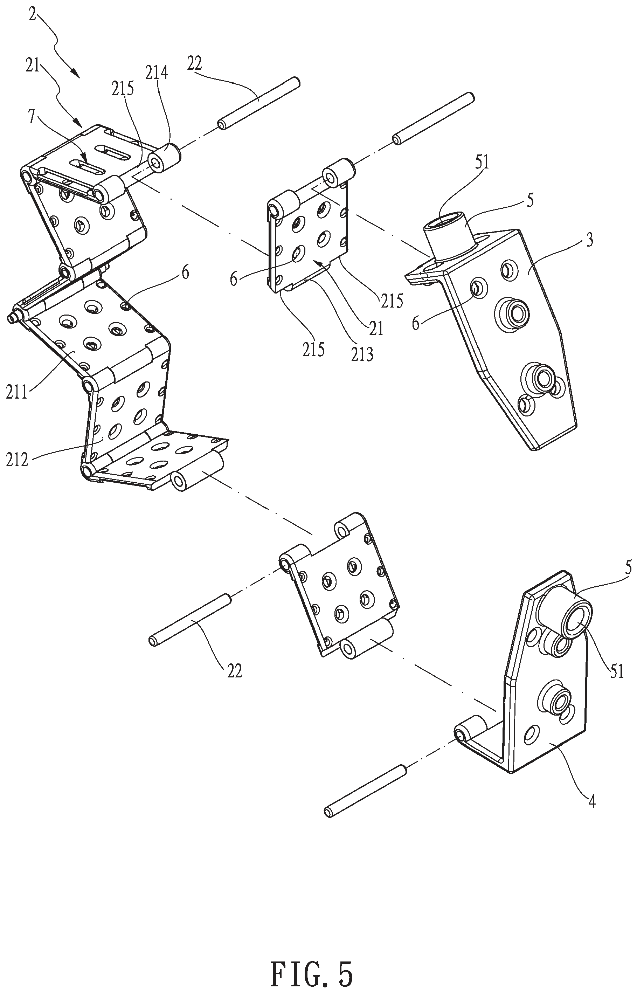

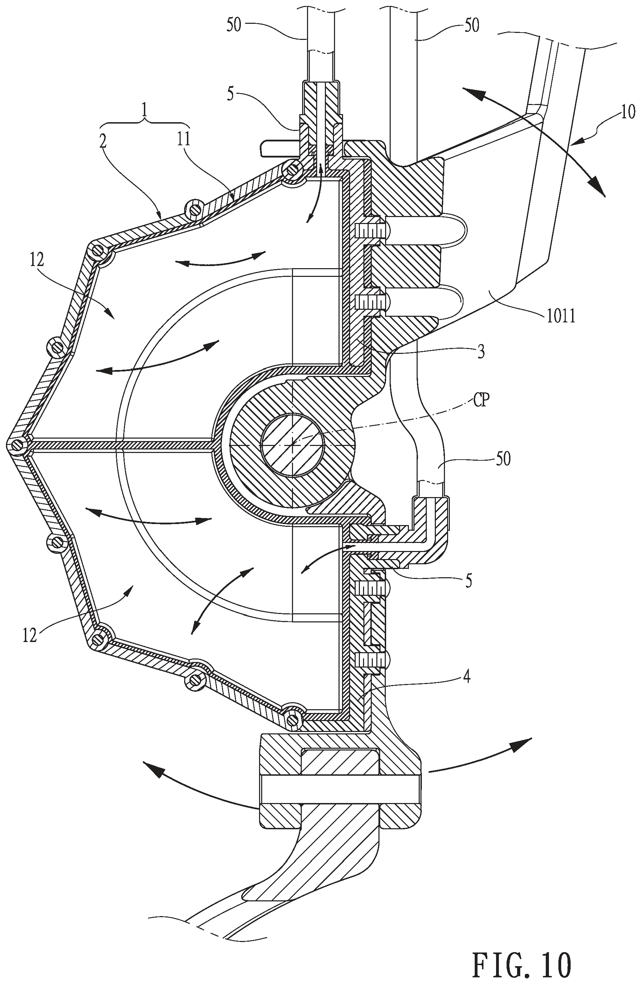

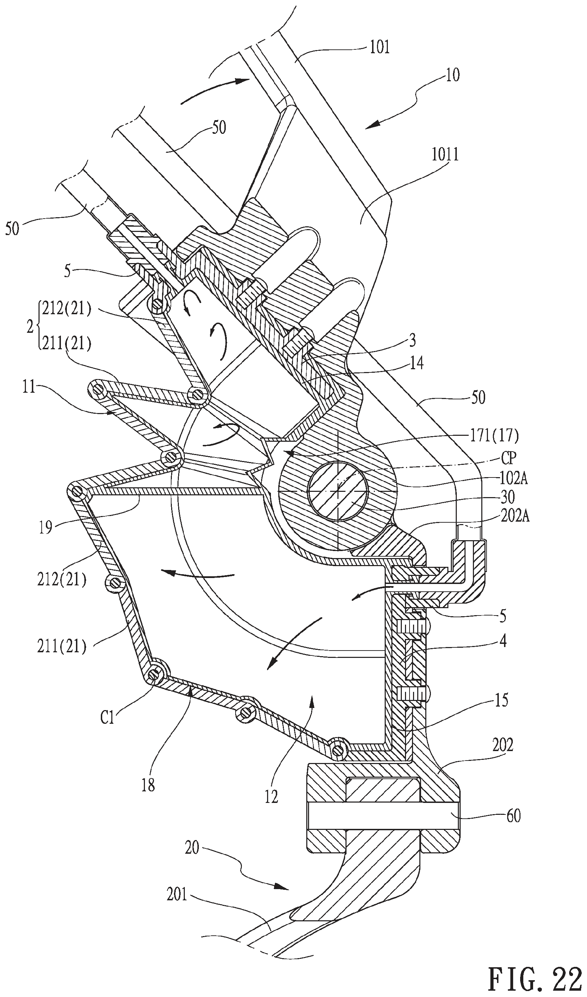

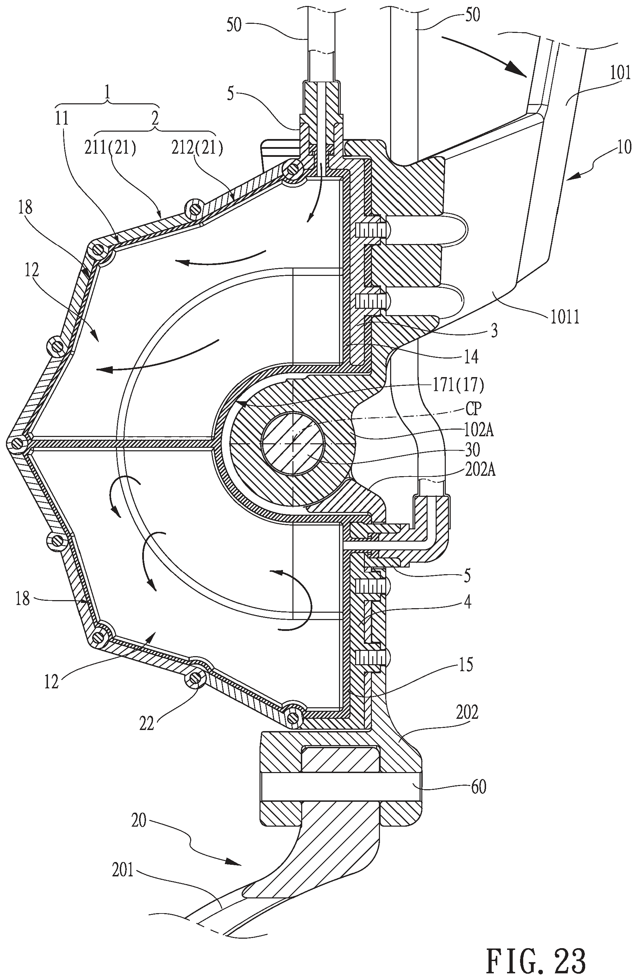

Referring to FIGS. 4 and 5, the present invention may further include a partition wall (19) disposed within the fluid chamber (12) between the two lateral faces (13A, 13B) of the bladder body (11). The partition wall (19) divides the originally single fluid chamber (12) into two fluid chambers (12) (with the fluid chambers (12) retaining the same reference numeral to avoid confusion, as only the quantity changes). Both the actuating link (3) and the restraining link (4) are each provided with a fluid connector (5), and each fluid connector (5) on the actuating link (3) and the restraining link (4) has a channel (51) communicating with a respective fluid chamber (12), thereby allowing fluid in each of the two fluid chambers (12) to flow in and out separately through the respective fluid connectors (5). During pressurization of the two fluid chambers (12) via the toggle links (21) of the toggle-link skeleton (2), the maximum thrust effect is generated when the included angle between the first toggle bar (211) and the second toggle bar (212) approaches or equals 180 degrees (see FIGS. 22 and 23). Accordingly, when the power-assist actuator (1) generates an assist-pushing action, the two fluid chambers (12) may be selected for staged pressurization to accelerate the response of the main arm body (101) to the assist-pushing thrust of the power-assist actuator (1). In addition, a conduit hole (1011A) is provided in the connecting portion (1011) of the thigh bracket (10) near the first fixing seat (102). When a fluid delivery conduit (50) is passed through the conduit hole (1011A), directional guidance and positioning are achieved. When fluid delivery conduits (50) are connected to both the fluid connector (5) on the actuating link (3) and the fluid connector (5) on the restraining link (4), all fluid delivery conduits (50) can be bundled and routed to the fluid supply system (40) (see FIGS. 1 and 10).

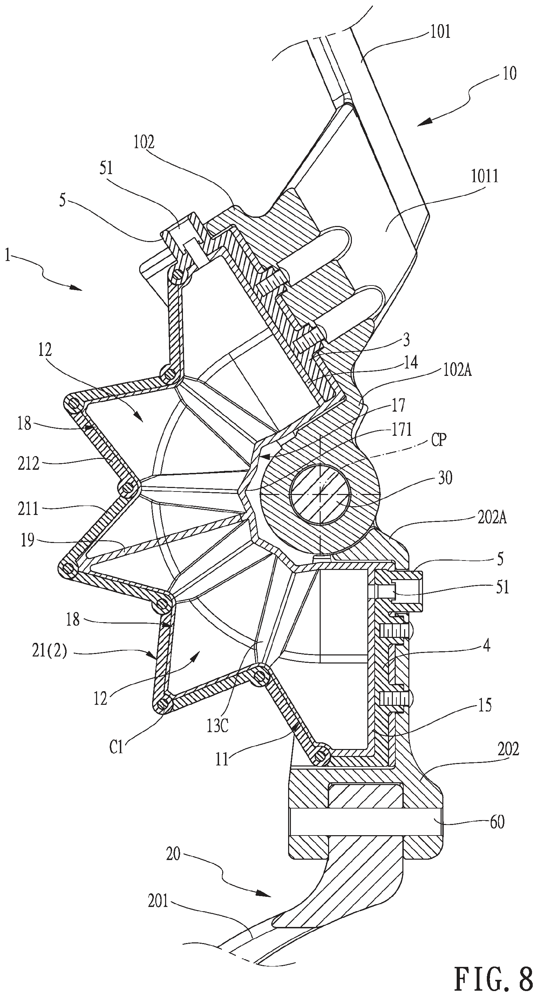

Referring to FIGS. 5 and 8, the toggle links (21) are pivotally connected to each other, with the first toggle bar (211) and the second toggle bar (212) of each toggle link (21) pivotally connected and capable of pivotal movement. The two ends of each first toggle bar (211) and each second toggle bar (212) are respectively formed with mutually mating male hinge hole portions (213) and female hinge hole portions (214), and a pivot pin (22) passes through each male hinge hole portion (213) and corresponding female hinge hole portion (214) to form the pivot connection. The mating end faces of each male hinge hole portion (213) and female hinge hole portion (214) are each further formed with a locking edge end (215). The V-shaped included angle between the first toggle bar (211) and the second toggle bar (212) of each toggle link (21) is not permitted to exceed 180 degrees. By limiting the V-shaped included angle between the first toggle bar (211) and the second toggle bar (212) of each toggle link (21) as it approaches 180 degrees, an abnormal thrust failure (outward flipping) of any toggle link (21) during the assist-pushing action of the power-assist actuator (1) can be prevented.

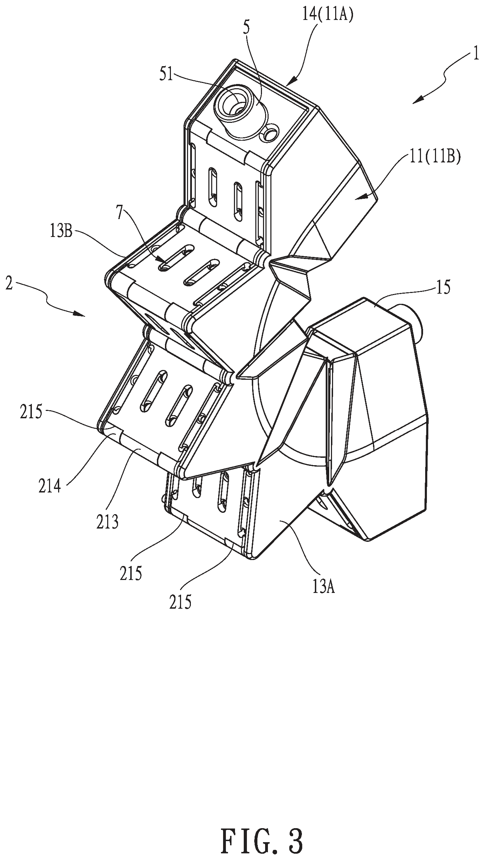

Referring to FIGS. 3 through 6, the bladder body (11) of the power-assist actuator (1) is constructed from a box-shaped bladder member (11A) and a cover member (11B) that are fitted together, with the non-open side faces of the cover member (11B) and the box-shaped bladder member (11A) serving as the two lateral faces (13A, 13B). The box-shaped bladder member (11A) and the cover member (11B) are sealed together to form the enclosed fluid chamber (12), wherein the actuation end (14), the restraint end (15), and each deformable section (18) are all provided on the box-shaped bladder member (11A). During mold-forming of the box-shaped bladder member (11A), the toggle-link skeleton (2) may be embedded in the mold so as to be integrally formed with the box-shaped bladder member (11A), enabling the toggle-link skeleton (2) to be bonded to the actuation end (14), the restraint end (15), and each deformable section (18). As shown in FIG. 5, the surfaces of the toggle links (21) of the toggle-link skeleton (2), the actuating link (3), and the restraining link (4) are each distributed with a plurality of material-penetrating holes (6) and a plurality of filler grooves (7), so that during mold-forming of the box-shaped bladder member (11A), the material can enter and pass through the material-penetrating holes (6) and fill the filler grooves (7) to form a composite bond, thereby making it difficult to separate the toggle-link skeleton (2) from the bladder body (11). Furthermore, the non-open side faces of the box-shaped bladder member (11A) and the cover member (11B) serve as the two lateral faces (13A, 13B), and a plurality of concave folds (13C) are formed on the surfaces of the two lateral faces (13A, 13B), each concave fold (13C) being recessed inwardly toward the fluid chamber (12). By providing the concave folds (13C), the bladder body (11) is capable of inward concave deformation and outward expansion deformation, preventing the two lateral faces (13A, 13B) from suffering over-stretching fatigue and tearing when the power-assist actuator (1) repeatedly generates cushioning and assist-pushing actions.

Referring to FIGS. 1 and 2, in order to further stabilize the power-assist actuator (1) during its deformation and actuation, the main arm body (101) of the thigh bracket (10) is an elongated arm having a concave surface (1012) on one side, which conforms to the user's thigh so that the main arm body (101) can be firmly secured to the thigh with straps. A plurality of strap apertures (1013) are formed on both sides of the main arm body (101) so that straps can pass through the strap apertures (1013) and simply bind the main arm body (101) to the thigh. The lower leg bracket (20) employs a second pivot shaft (60) to pivotally connect the upper end of the restraining arm (201) to the bottom end of the second fixing seat (202). When the user is wearing the knee joint brace and walking normally, the restraining arm (201) is permitted to swing laterally at the bottom end of the second fixing seat (202), so that the movement of the user's lower leg is not unnecessarily or excessively restricted or limited by the restraining arm (201). A supporting rest portion (201A) formed at the bottom end of the restraining arm (201) of the lower leg bracket (20) is an arcuate frame member adapted to rest against the back of the user's lower leg, with strap holes (201B) provided at each end thereof, through which straps may be used to non-fixedly secure the supporting rest portion (201A) to the user's lower leg, with the straps restraining the supporting rest portion (201A) from moving away from the lower leg.

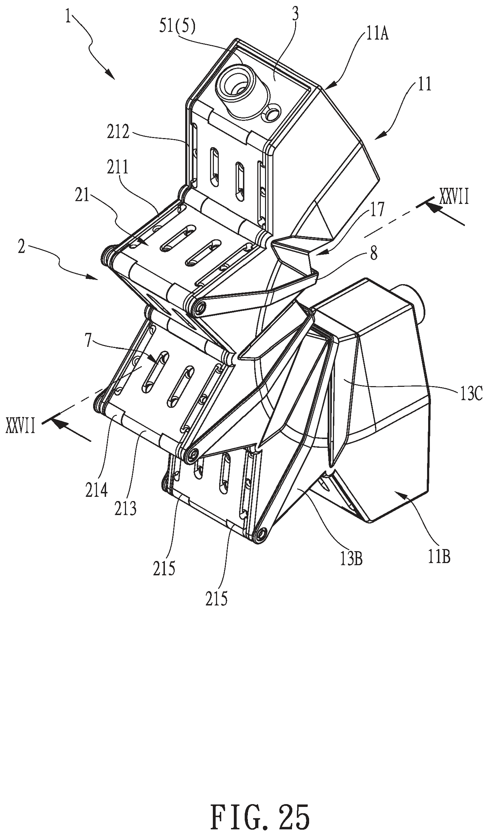

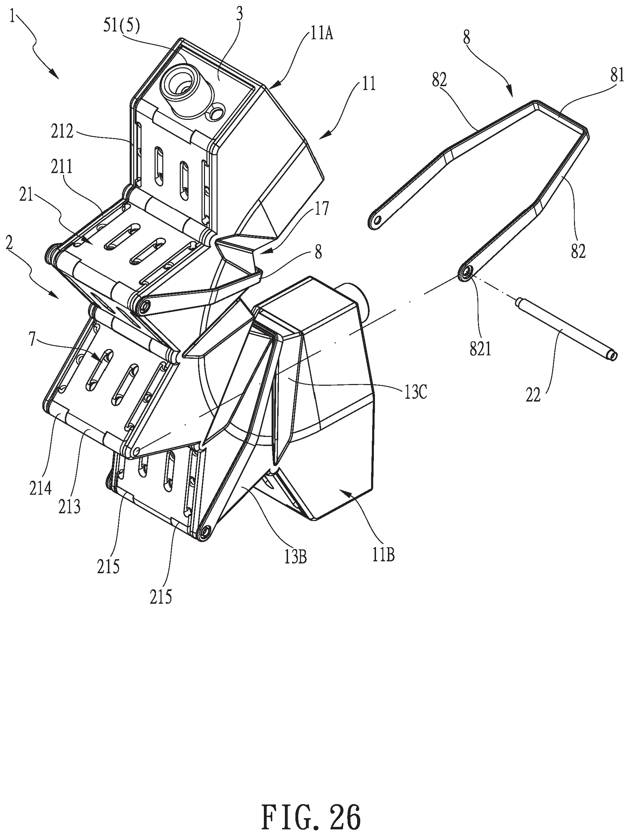

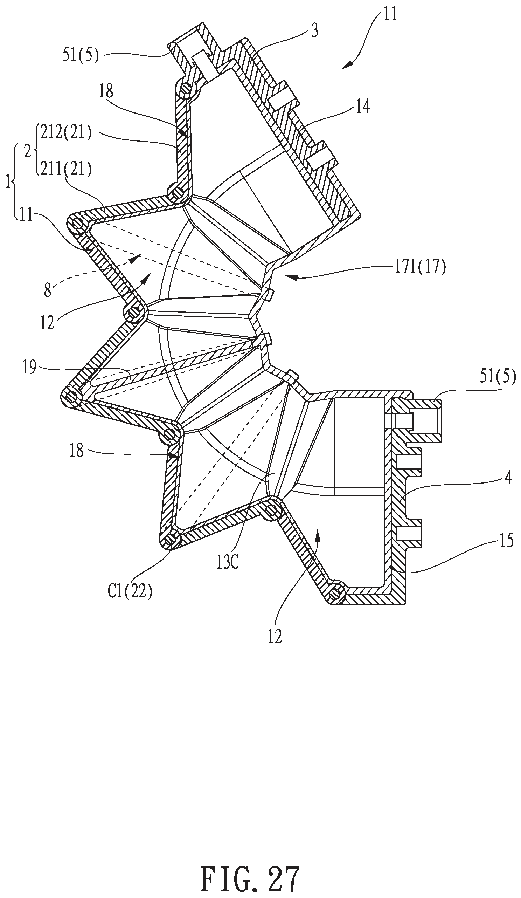

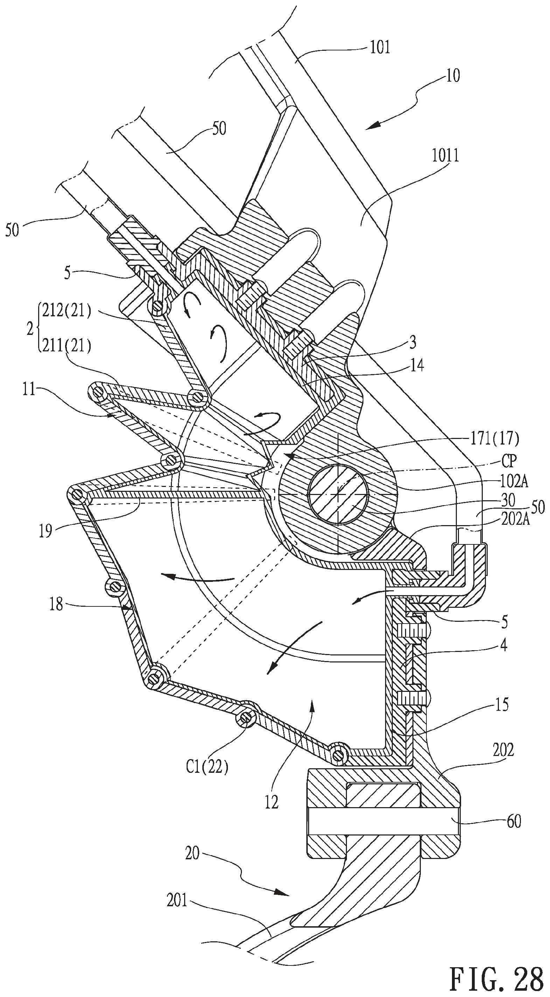

Regarding the structural features of the toggle-link skeleton (2) and referring to FIGS. 25 through 28, in order to reliably interlock and drive the bladder body (11), the toggle links (21) of the toggle-link skeleton (2) are pivotally connected to each other by pivot pins (22), with the pivot connection points of the pivot pins (22) defined as toggle pivot points (C1). The two ends of the pivot pin (22) at each toggle pivot point (C1) extend through the two lateral faces (13A, 13B), and a U-shaped retainer (8) is fixedly secured to each pivot pin. Each U-shaped retainer (8) comprises a bearing bar (81) and a pair of arm bars (82) extending from both ends of the bearing bar (81). A fixing hole (821) is provided at the front end of each arm bar (82), and each fixing hole (821) is assembled onto a respective end of the pivot pin (22) (which may be secured by riveting), so that each pair of arm bars (82) is overlaid on the surfaces of the two lateral faces (13A, 13B) while the bearing bar (81) supports the inner peripheral surface (17), permanently fixing the distance between each toggle pivot point (C1) and the center point (CP). By preventing the distance between the toggle pivot point (C1) and the center point (CP) from changing when the fluid chamber (12) within the bladder body (11) expands, each toggle pivot point (C1) acts as a support point at each end of the respective toggle link (21) during the assist-pushing action of the power-assist actuator (1), thereby facilitating the smooth opening of the included angle between the first toggle bar (211) and the second toggle bar (212) of each toggle link (21), and thus effectively generating a stable thrust force concentrated toward the actuating link (3).

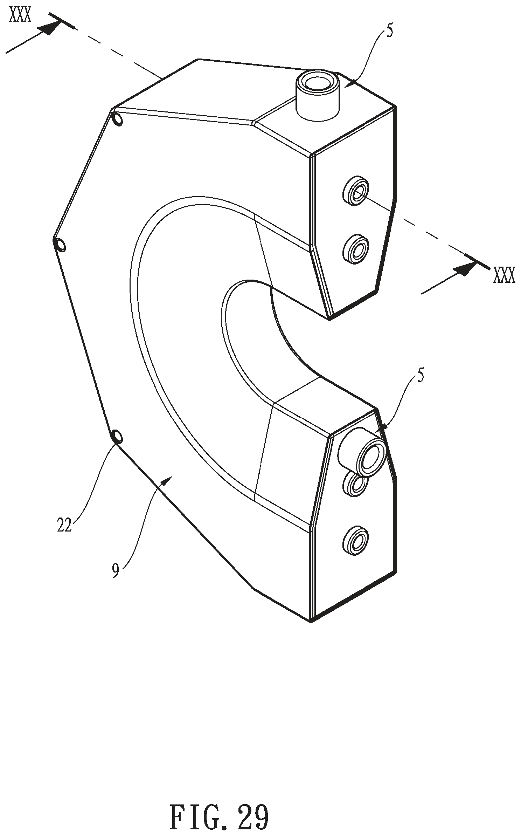

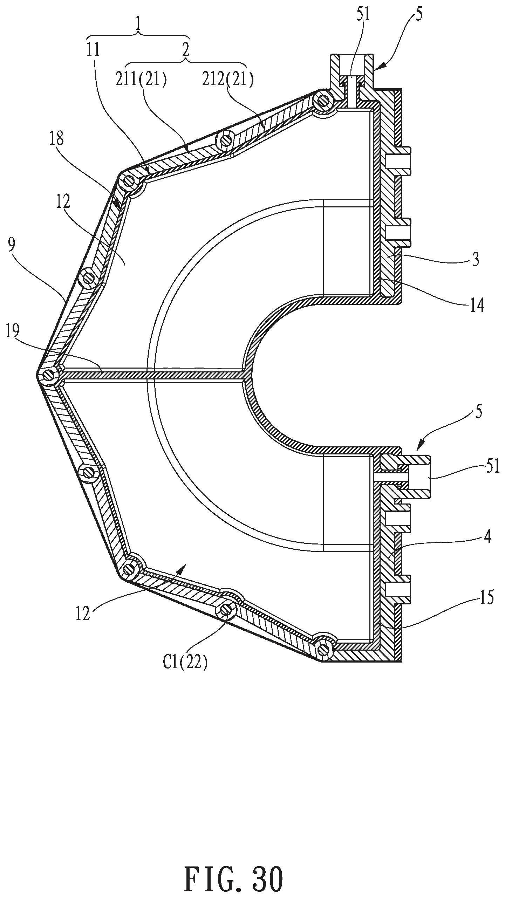

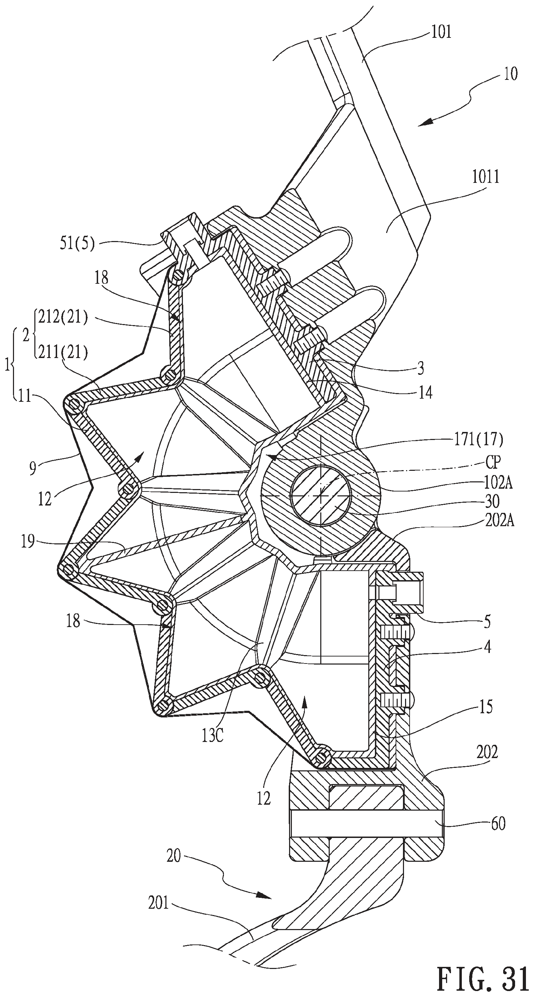

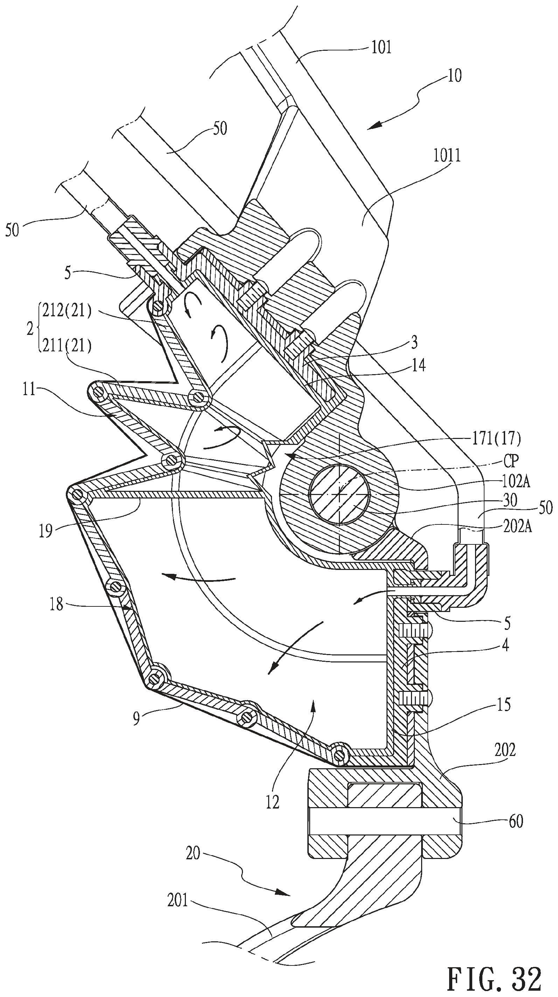

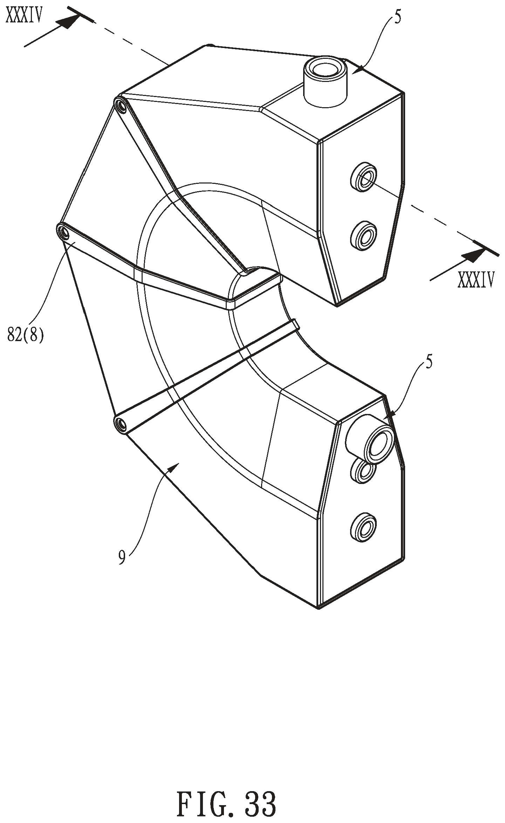

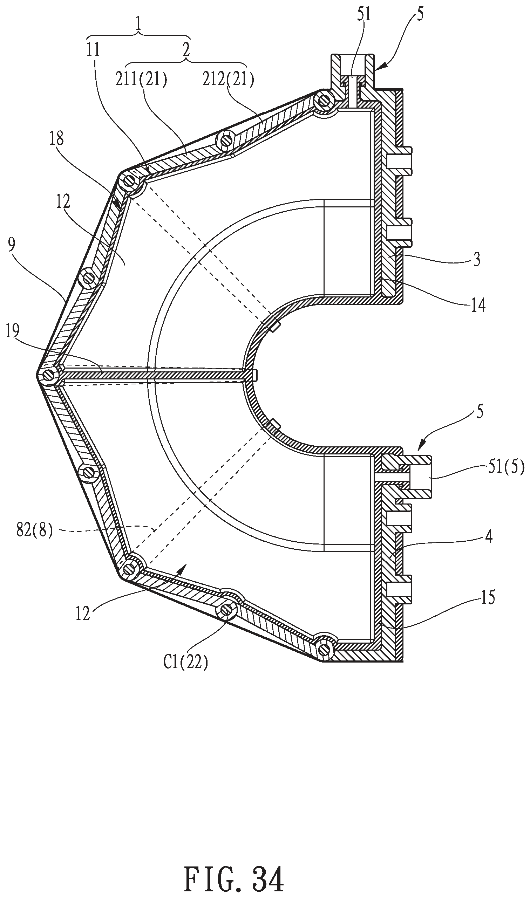

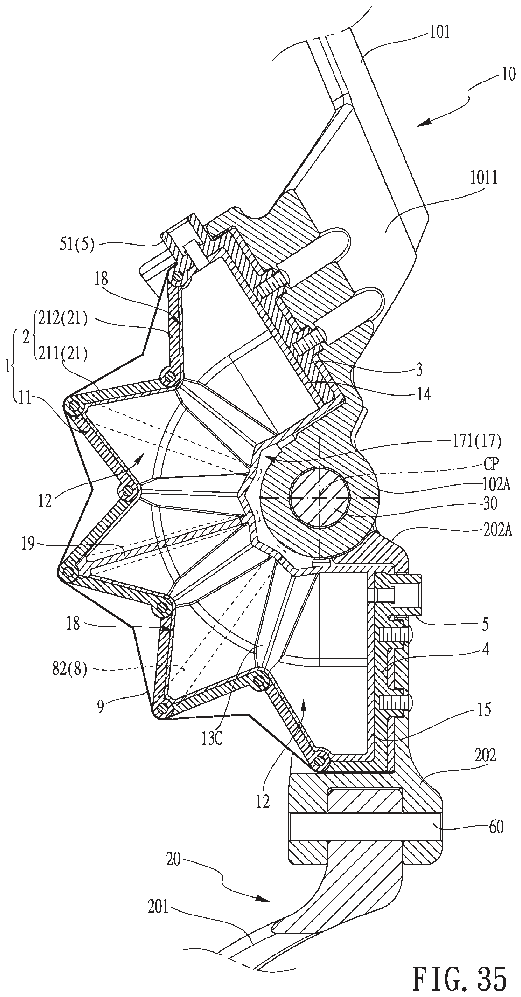

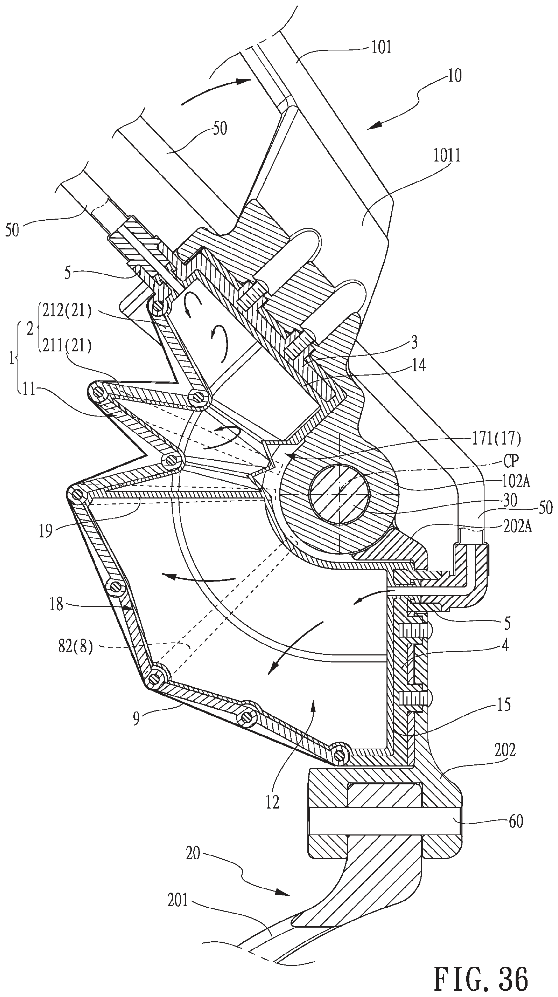

Finally, based on the foregoing embodiments and referring to FIGS. 29 through 36, the present invention may further include a fabric sleeve (9) covering the surface of the power-assist actuator (1). The fabric sleeve (9) may be a sleeve formed from a flexible, tear-resistant material (such as natural or synthetic fiber woven fabric). The fabric sleeve (9) is provided to limit the shape of the bladder body (11) during expansion, restricting the opening angle between the first toggle bar (211) and the second toggle bar (212) to not exceed 180 degrees, so that during the assist-pushing action of the power-assist actuator (1), each toggle link (21) delivers a stable and effective thrust force concentrated onto the actuating link (3). The toggle links (21) are pivotally connected by pivot pins (22), with the pivot connection points defined as toggle pivot points (C1), the two ends of the pivot pin (22) at each toggle pivot point (C1) extend through the two lateral faces (13A, 13B) and the fabric sleeve (9). A plurality of U-shaped retainers (8) are further mounted around each pivot pin (22), with each pair of arm bars (82) overlaid on the surface of the fabric sleeve (9) and the bearing bar (81) supporting the inner peripheral surface (17), permanently fixing the distance between each toggle pivot point (C1) and the center point (CP). When the one or more fluid chambers (12) within the bladder body (11) expand, the distance between each toggle pivot point (C1) and the center point (CP) does not change. During the assist-pushing action of the power-assist actuator (1), with each toggle pivot point (C1) serving as a support point at each end of the respective toggle link (21) and in conjunction with the fabric sleeve (9) limiting the included angle between the first toggle bar (211) and the second toggle bar (212) to not exceed 180 degrees, the first toggle link (21) can generate a smoother and more effective thrust force concentrated onto the actuating link (3).

While we have shown and described the embodiment in accordance with the present invention, it should be clear to those skilled in the art that further embodiments may be made without departing from the scope of the present invention.

## Claims

1. A toggle-link knee joint support and assist brace, comprising:
a thigh bracket (10) having a main arm body (101) and a first fixing seat (102) formed at a bottom end of the thigh bracket (10), the thigh bracket (10) adapted to be securable to a human thigh;
a lower leg bracket (20) having a restraining arm (201) and a second fixing seat (202) formed at an upper end of the lower leg bracket (20), the lower leg bracket (20) adapted to be supportable against a human lower leg;
a pivot shaft (30) interconnecting the first fixing seat (102) and the second fixing seat (202); and
a power-assist actuator (1) disposed between the first fixing seat (102) and the second fixing seat (202);
wherein the power-assist actuator (1) comprises:
a flexible and fluid-tight bladder body (11), an interior of the bladder body (11) defining at least one fluid chamber (12), the bladder body (11) having an actuation end (14) and a restraint end (15) located respectively at opposite ends thereof, the bladder body (11) forming a plurality of deformable sections (18) between the actuation end (14) and the restraint end (15), the deformable sections (18) being interconnected and allowing the at least one fluid chamber (12) to expand or contract as fluid flows in or out;
a toggle-link skeleton (2) disposed on the exterior of the bladder body (11), the toggle-link skeleton (2) comprising a plurality of mutually pivoted toggle links (21), each toggle link (21) being formed by pivotally connecting a first toggle bar (211) and a second toggle bar (212), each first toggle bar (211) being pivotally connected to an adjacent second toggle bar (212), and each first toggle bar (211) and each second toggle bar (212) corresponding to a respective deformable section (18) so as to support and guide the deformation of the bladder body (11);
an actuating link (3) and a restraining link (4), the actuating link (3) connecting one end of the toggle-link skeleton (2) and being coupled to the first fixing seat (102), the restraining link (4) connecting the other end of the toggle-link skeleton (2) and being coupled to the second fixing seat (202); and
at least one fluid connector (5) disposed on the actuating link (3) or the restraining link (4) and in fluid communication with the at least one fluid chamber (12) of the bladder body (11), whereby when the bladder body (11) expands or contracts under fluid pressure, a deformation is converted, through the toggle-link skeleton (2), into a supporting or assist-pushing force between the first fixing seat (102) and the second fixing seat (202).

2. The toggle-link knee joint support and assist brace as claimed in claim 1, wherein the at least one fluid chamber (12) of the bladder body (11) includes a partition wall (19) dividing the at least one fluid chamber (12) into two fluid chambers (12), the actuating link (3) and the restraining link (4) are each provided with a fluid connector (5), each fluid connector (5) communicates with a respective fluid chamber (12), and fluid in each of the two fluid chambers (12) flows in and out separately through the respective fluid connectors (5).

3. The toggle-link knee joint support and assist brace as claimed in claim 2, wherein the main arm body (101) of the thigh bracket (10) is provided with a conduit hole (1011A) in proximity to the first fixing seat (102) for routing of fluid conduits therethrough.

4. The toggle-link knee joint support and assist brace as claimed in claim 1, wherein each first toggle bar (211) is pivotally connected to a corresponding second toggle bar (212) by a pivot pin (22) passing through respective pivot connection points, so that each first toggle bar (211) and each second toggle bar (212) are pivotable, a V-shaped included angle between each first toggle bar (211) and each corresponding second toggle bar (212) does not exceed 180 degrees.

5. The toggle-link knee joint support and assist brace as claimed in claim 1, wherein the bladder body (11) is assembled from a box-shaped bladder member (11A) and a cover member (11B) that are fitted and sealed together to define the at least one fluid chamber (12) for receiving fluid.

6. The toggle-link knee joint support and assist brace as claimed in claim 5, wherein surfaces of the toggle-link skeleton (2), each toggle link (21), the actuating link (3), and the restraining link (4) are each formed with at least one material-penetrating hole (6) and at least one filler groove (7).

7. The toggle-link knee joint support and assist brace as claimed in claim 5, wherein a lateral face surface of the bladder body (11) are formed with a plurality of concave folds (13C) directed toward the at least one fluid chamber (12) to control an expansion profile in a predetermined direction during expansion.

8. The toggle-link knee joint support and assist brace as claimed in claim 1, wherein the main arm body (101) is an elongated arm having a concave surface (1012) formed on one side thereof.

9. The toggle-link knee joint support and assist brace as claimed in claim 8, wherein two side surfaces of the main arm body (101) are formed with a plurality of strap apertures (1013).

10. The toggle-link knee joint support and assist brace as claimed in claim 1, further comprising a second pivot shaft (60) pivotally connecting the restraining arm (201) and the second fixing seat (202) such that the restraining arm (201) is rotatable relative to the second fixing seat (202).

11. The toggle-link knee joint support and assist brace as claimed in claim 1, wherein a lower end of the restraining arm (201) of the lower leg bracket (20) is formed with a supporting rest portion (201A) that is arcuate in shape so as to conform to a curved surface of a human lower leg, and strap holes (201B) are respectively provided at two ends of the supporting rest portion (201A).

12. The toggle-link knee joint support and assist brace as claimed in claim 1, wherein a toggle pivot point (C1) of each toggle link (21) is connected by a pivot pin (22), and a plurality of U-shaped retainers (8) are provided and respectively assembled onto each pivot pin (22), each U-shaped retainer (8) spans over an exterior of the bladder body (11) and is configured to support an expandable portion of the bladder body (11) and to restrain a distance between the toggle pivot point (C1) of each toggle link (21) and a center point of the bladder body (11).

13. The toggle-link knee joint support and assist brace as claimed in claim 1, further comprising a fabric sleeve (9) disposed over an exterior of the toggle-link skeleton (2), the fabric sleeve (9) being made of a flexible and tear-resistant material and configured to limit an extent of deformation of the bladder body (11) during expansion.

14. The toggle-link knee joint support and assist brace as claimed in claim 13, wherein a toggle pivot point (C1) of each toggle link (21) is connected by a pivot pin (22), each pivot pin (22) extends through the fabric sleeve (9) disposed on the exterior of the toggle-link skeleton (2), a plurality of U-shaped retainers (8) are provided and respectively assembled onto each pivot pin (22), each U-shaped retainer (8) spans over an exterior of the fabric sleeve (9) and is configured to support the bladder body (11) and to restrain a distance between the toggle pivot point (C1) of each toggle link (21) and a center point (CP) of the bladder body (11).
